# EUROPÄISCHE PATENTSCHRIFT

(11) **EP 0 543 345 B1**
(45) Veröffentlichungstag und Bekanntmachung des Hinweises auf die Patenterteilung: **07.09.1994**
(21) Anmeldenummer: 92119636.6
(22) Anmeldetag: 17.11.1992
(51) Int. Cl.: C07D 311/58, C07C 39/373

(54) **Substituierte Pentaalkylchromane**
Substituted pentaalkylchromanes
Pentaalkylchromanes substitués

(30) Priorität: 20.11.1991 CH 3393/91
(43) Veröffentlichungstag der Anmeldung: 26.05.1993
(73) Patentinhaber: LONZA AG, CH-3945 Gampel/Wallis (CH); Sankyo Company Limited, Tokyo (JP)
(72) Erfinder: Laffan, David, Dr., Visp (Kanton Wallis) (CH)
(74) Vertreter: Weinhold, Peter, Dr.

(56) Entgegenhaltungen:
- EP-A- 0 036 160
- GB-A- 2 055 097

## Beschreibung

Die Erfindung betrifft substituierte Pentaalkylchromane der allgemeinen Formel 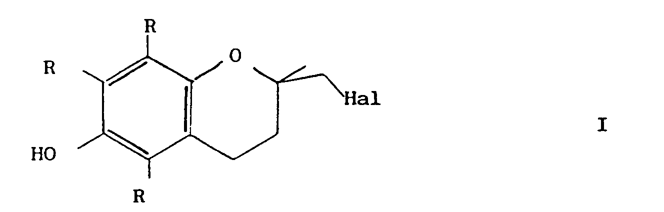 worin R eine Niederalkylgruppe mit 1-4 C-Atomen und Hal, Chlor, Brom oder Jod bedeuten, sowie ein Verfahren zu deren Herstellung. Pentaalkylchromane, insbesondere die Tetramethylderivate der allgemeinen Formel 1,sind wertvolle Zwischenprodukte zur Herstellung von z.B. lipidsenkenden Pharmazeutika (J.Med.Chem. 1989 32, S.421). Zur Herstellung der genannten Pharmazeutika war es bekannt, als Zwischenprodukte, Hydroxyalkylchromane der allgemeinen Formel 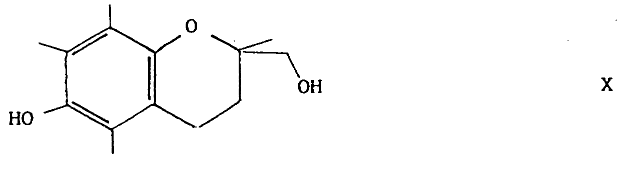 einzusetzten (J.Med.Chem. 1989, 32, S.421).

Diese Hydroxyalkylchromane sind gemäss DE-OS 30 10 504 aus dem entsprechenden Hydrochinon und dem entsprechenden Butendiol nur mit einer Ausbeute von 10% erhältlich.

Gemäss DE-OS 23 64 165 ist es auch bekannt, die Hydroxyalkylchromane in einer mehrstufigen Synthese herzustellen. Diese Synthese ist aber sehr aufwendig und für die Umsetzung in die Technik nicht geeignet.

Die Aufgabe bestand daher darin, einen neuen Zugang zu den genannten Pharmazeutika zu entwickeln, welcher die Nachteile der bekannten Verfahren und Zwischenprodukte nicht aufweist.

Mit den subst. Pentaalkylchromanen gemäss Patentanspruch 1, sowie mit einem einfachen Verfahren zu deren Herstellung konnte die Aufgabe gelöst werden.

Im ersten Schritt wird erfindungsgemäss ein Trialkylhydrochinon der allgemeinen Formel 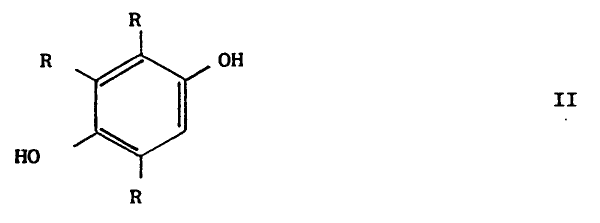 worin R die genannte Bedeutung hat, mit halogenierten Butenolen der Formel 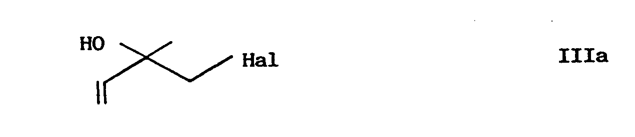 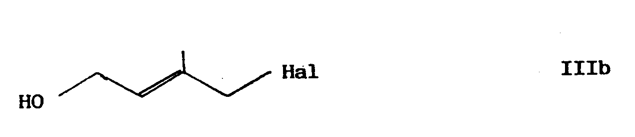 worin Hal die genannte Bedeutung hat,in Gegenwart einer Lewis-Säure so umgesetzt, dass als Zwischenprodukt ein Tetraalkylhydrochinon der allgemeinen Formel 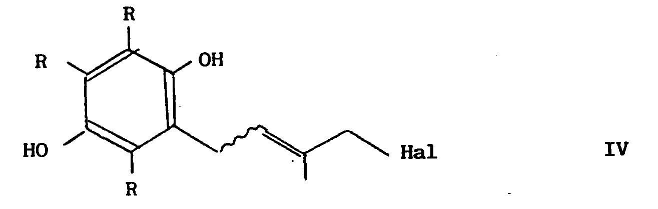
worin R und hal die genannten Bedeutungen haben resultiert. Diese Verbindungen sing bisher nicht beschrieben und ebenfalls Bestandteil der Erfindung.

Bevorzugt wird Trimethylhydrochinon (Formel II mit R = CH₃) mit 1-Chlor-2-methylbut-3-en-2-ol(Formel Illa mit Hal = CI) zum entsprechenden Tetraalkylhydrochinon (Formel IV mit R = CH₃ und Hal = CI) umgesetzt.

Als Lewis-Säure wird zweckmässig Eisenchlorid, Zinntrifluormethansulfonat oder Bortrifluorid bzw. eine seiner Komplexverbindungen, bevorzugt Bortrifluorid oder eine seiner Komplexverbindungen, wie z.B. Bortrifluorid-etherat eingesetzt. Die Lewis-Säure wird zweckmässig in einer Menge von 1 mol bis 5 mol bezogen auf das eingesetzte Trialkylhydrochinon, eingesetzt

Von Vorteil wird in Gegenwart eines inerten Lösungsmittels wie z.B. Toluol oder halogenierten Kohlenwasserstoffen, wie Methylenchlorid, bei einer Temperatur zwischen -10_{°}C und 50 _{°} C, vorzugsweise bei Raumtemperatur gearbeitet.

Bevorzugt wird so verfahren, dass unmittelbar dann, wenn im Reaktionsgemisch praktisch kein Ausgangsprodukt (Trialkylhydrochinon) mehr nachweisbar ist, die Reaktion abgebrochen und das resultierende Tetraalkylhydrochinon isoliert wird.

In der Folgestufe wird das Tetraalkylhydrochinon der allgemeinen Formel IV in Gegenwart einer starken Säure zum Endprodukt der allgemeinen Formel I cyclisiert.

Als starke Säure findet zweckmässig eine starke Brönsted-Säure, wie z.B. Trifluormethansulfonsäure, Chlorwasserstoff oder Fluorsulfonsäure Anwendung.

Diese Brönsted-Säuren werden zweckmässig in einer Menge von 0,05 mol bis 2 mol, bezogen auf das Tetraalkylhydrochinon, eingesetzt.

Von Vorteil wird die Cyclisierung in Gegenwart eines unpolaren Lösungsmittels wie z.B. Toluol, Tetrachlorkohlenstoff oder Hexan bei einer Temperatur zwischen -10°C und 70°C, vorzugsweise bei Raumtemperatur durchgeführt.

Die resultierenden subst. Pentaalkylchromane der allgemeinen Formel 1 sind bisher nicht beschrieben. Bevorzugtes Derivat ist das 2-Chlormethyl-6-hydroxy-2,5,7,8-tetramethylchroman mit R = CH₃ und Hal = CI.

### Beispiel

### a) Verfahren zur Herstellung von 2-(1-Chlor-2-methylbut-2-en-4-yl)-1,4-dihydroxy-3,5,6-trimethylbenzol

Trimethylhydrochinon (2,00 g, 14 mmol) wurde in einer Mischung von Chlorbenzol (10 ml) und Hexan (5 ml) bei Raumtemperatur aufgeschlämmt. Bortrifluorid-diethylether-Komplex (3,60 ml einer 48%-igen Lösung von Bortrifluorid in Diethylether, (1,95 g Bortrifluorid, 28 mmol) wurde dann während 15 min zugegeben. Anschliessend wurde dann das 1-Chlor-2-methylbut-3-en-2-ol (2,42 g, 20 mmol) während 15 min zugetropft. Nach 30 min wurde die Reaktionsmischung filtriert, das Filtrat mit Chlorbenzol (10 ml) gewaschen und bei 40° C/20 mbar wärend 8 h getrocknet. Es wurden 2,24 g (62,6%) 2-(1-Chlor-2-methylbut-2-en-4-yl)-1,4-dihydroxy-3,5,6-trimethylbenzol erhalten. Schmelzpunkt: 118-122 ° C (Zersetzung) ¹H-NMR: (CDCl₃, 300 MHz) δ in ppm: 5,50 (t, 1 H, J=7,5Hz);
4,02 (s, 2H);
3,45 (d, 2H, J = 7,5Hz);
2,17 (s, 6H);
2,15 (s, 3H);
1,95 (s, 3H)
Isomer: 5,48 (t, 1 H, J = 7,5Hz);
4,02 (s, 2H);
3,48 (d, 2H, J=7,5Hz);
2,17 (s, 6H);
2,15 (s, 3H);
1,95 (s, 3H)

### b) Verfahren zur Herstellung von 2-Chlormethyl-6-hydroxy-2,5,7,8-tetramethylchroman

2-(1-Chlor-2-methylbut-2-en-4-yl)-1,4-dihydroxy-3,5,6-trimethylbenzol (4,2 g, 16 mol) wurden in Tetrachlorkohlenstoff (200 ml) aufgeschlämmt. Trifluormethansulfonsäure (1,44 ml, 2,44 g, 16 mmol) wurde dann zugegeben und die Reaktionsmischung gerührt. Nach 30 min wurde Wasser (100 ml) zugegeben und die Mischung mit Methylenchlorid (100 ml) extrahiert. Die organische Phase wurde getrocknet (MgS0₄) und eingeengt. Dies ergab rohes 2-Chlormethyl-6-hydroxy-2,5,7,8-tetramethylchroman (5,3 g). Umkristallisation aus Hexan (40 ml) ergab 2,7 g (64%) reines 2-Chlormethyl-6-hydroxy-2,5,7,8-tetramethylchroman. Schmelzpunkt: 77-82 ° C. ¹H-NMR: (CDC1₃, 300 MHz) δ in ppm: 4,25 (s, 1 H);
3,53 (d, 1 H, J = 12,OHz);
3,50 (d, 1 H, J = 12,OHz);
2,62 (t, 2H, J=7,OHz);
2,17 (s, 3H);
2,11 (s, 6H);
2,10-2,02 (m, 1 H);
1,90-1,78 (m, 1 H);
1,40 (s, 3H)

## Patentansprüche (Patentansprüche für folgende(n) Vertragsstaat(en) : AT, BE, CH, LI, DE, DK, FR, GB, IE, IT, LU, NL, PT, SE)

1. Substituierte Pentaalkylchromane der allgemeinen Formel 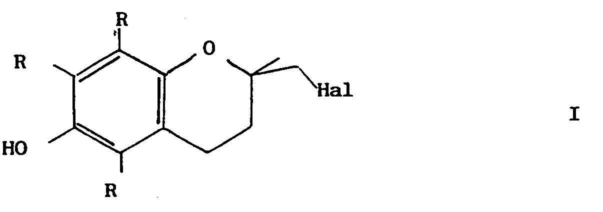 worin R eine Niederalkylgruppe mit 1 bis 4 C-Atomen und Hal Chlor, Brom oder Jod bedeutet.

2. 2-Chlormethyl-6-hydroxy-2,5,7,8-tetramethylchroman der Formel

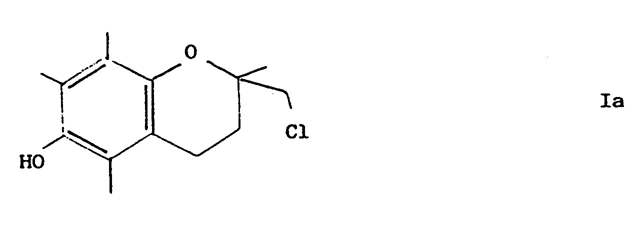

3. Verfahren zur Herstellung von substituierten Pentaalkylchromanen der allgemeinen Formel 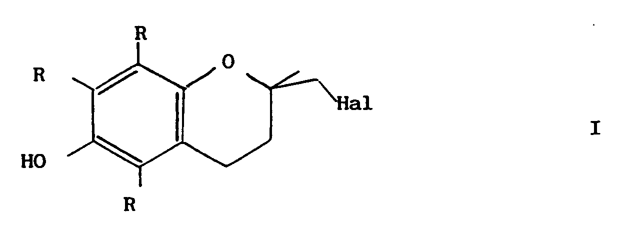 worin R eine Niederalkylgruppe mit 1 bis 4 C-Atomen und Hal, Chlor, Brom oder Jod bedeutet, dadurch gekennzeichnet, dass ein Trialkylhydrochinon der allgemeinen Formel 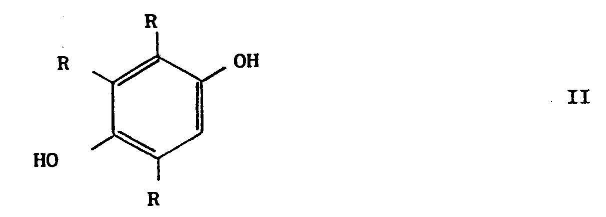 worin R die genannte Bedeutung hat, mit einem halogenierten Butenol der Formel 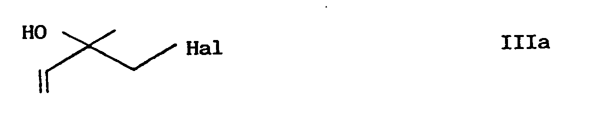 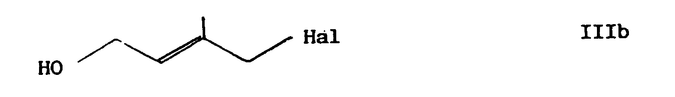 worin Hal die genannte Bedeutung hat, in Gegenwart einer Lewis-Säure so umgesetzt wird, dass ein Tetraalkylhydrochinon der allgemeinen Formel 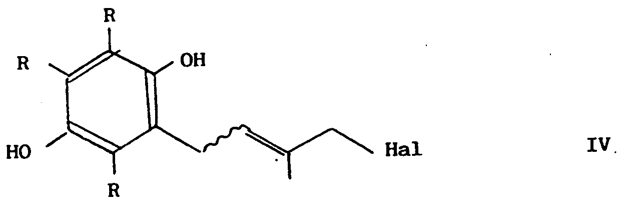 worin R und Hal die genannte Bedeutung haben, resultiert, und das Tetraalkylhydrochinon schliesslich in Gegenwart einer starken Säure zum Endprodukt cyclisiert wird.

4. Verfahren nach Patentanspruch 3, dadurch gekennzeichnet, dass als Trialkylhydrochinon der allgemeinen Formel 11 das Trimethylderivat mit R = CH₃ und als halogeniertes Butenolderivat das 1-Chlor-2-methyl-but-3-en-2-ol der Formel Illa mit Hal = CI eingesetzt wird.

5. Verfahren nach Patentanspruch 3 oder 4, dadurch gekennzeichnet, dass als Lewis-Säure Bortrifluorid oder eine seiner Komplexverbindungen eingesetzt wird.

6. Verfahren nach einem der Patentansprüche 3 bis 5, dadurch gekennzeichnet, dass die Umsetzung zum Tetraalkylhydrochinon bei einer Temperatur zwischen -10°C und 50 °C in Gegenwart eines inerten Lösungsmittels durchgeführt wird.

7. Verfahren nach einem der Patentansprüche 3 bis 6, dadurch gekennzeichnet, dass die Umsetzung zum Tetraalkylhydrochinon so durchgeführt wird, dass unmittelbar dann, wenn im Reaktionsgemisch das Ausgangsprodukt Trialkylhydrochinon praktisch nicht mehr nachweisbar ist, die Reaktion abgebrochen und das resultierende Tetraalkylhydrochinon isoliert wird.

8. Verfahren nach einem der Patentansprüche 3 bis 7, dadurch gekennzeichnet, dass als starke Säure für die Cyclisierung eine starke Brönsted-Säure verwendet wird.

9. Verfahren nach einem der Patentansprüche 3 bis 8, dadurch gekennzeichnet, dass die Cyclisierung in einem unpolaren Lösungsmittel bei einer Temperatur zwischen -10°C und 70 °C durchgeführt wird.

10. Substituierte Tetraalkylhydrochinone der allgemeinen Formel 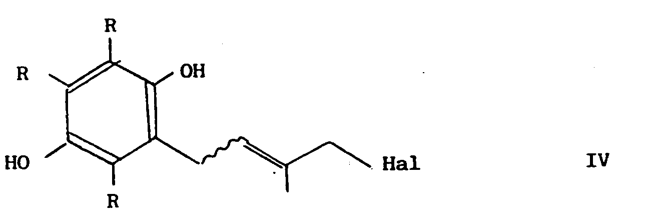 worin R und Hal die genannte Bedeutung haben.

11. 2-(1-Chlor-2-methylbut-2-en-4-yl)-1,4-dihydroxy-3,5,6-trimethylbenzol

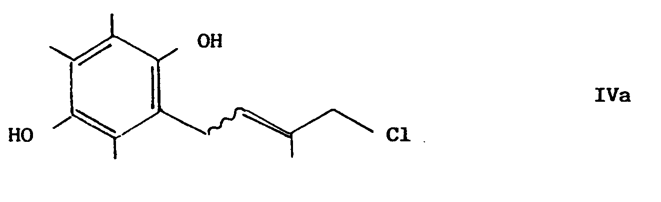

## Patentansprüche (Patentansprüche für folgende(n) Vertragsstaat(en) : ES, GR)

1. Verfahren zur Herstellung von substituierten Pentaalkylchromanen der allgemeinen Formel 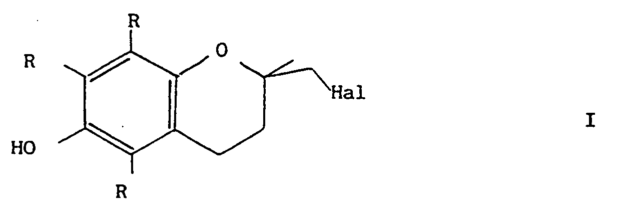 worin R eine Niederalkylgruppe mit 1 bis 4 C-Atomen und Hal, Chlor, Brom oder Jod bedeutet, dadurch gekennzeichnet, dass ein Trialkylhydrochinon der allgemeinen Formel 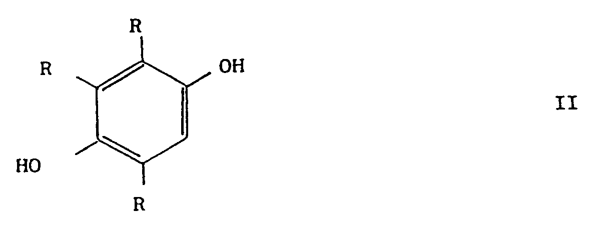 worin R die genannte Bedeutung hat, mit einem halogenierten Butenol der Formel 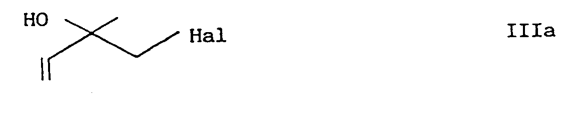 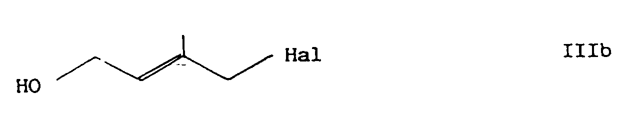 worin Hal die genannte Bedeutung hat, in Gegenwart einer Lewis-Säure so umgesetzt wird, dass ein Tetraalkylhydrochinon der allgemeinen Formel 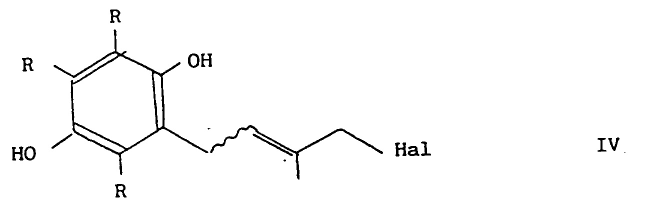 worin R und Hal die genannte Bedeutung haben, resultiert, und das Tetraalkylhydrochinon schliesslich in Gegenwart einer starken Säure zum Endprodukt cyclisiert wird.

2. Verfahren nach Patentanspruch 1, dadurch gekennzeichnet, dass als Trialkylhydrochinon der allgemeinen Formel 11 das Trimethylderivat mit R = CH₃ und als halogeniertes Butenolderivat das 1-Chlor-2-methyl-but-3-en-2-ol der Formel Illa mit Hal = CI eingesetzt wird.

3. Verfahren nach Patentanspruch 1 oder 2, dadurch gekennzeichnet, dass als Lewis-Säure Bortrifluorid oder eine seiner Komplexverbindungen eingesetzt wird.

4. Verfahren nach einem der Patentansprüche 1 bis 3, dadurch gekennzeichnet, dass die Umsetzung zum Tetraalkylhydrochinon bei einer Temperatur zwischen -10 _{°} C und 50 °C in Gegenwart eines inerten Lösungsmittels durchgeführt wird.

5. Verfahren nach einem der Patentansprüche 1 bis 4, dadurch gekennzeichnet, dass die Umsetzung zum Tetraalkylhydrochinon so durchgeführt wird, dass unmittelbar dann, wenn im Reaktionsgemisch das Ausgangsprodukt Trialkylhydrochinon praktisch nicht mehr nachweisbar ist, die Reaktion abgebrochen und das resultierende Tetraalkylhydrochinon isoliert wird.

6. Verfahren nach einem der Patentansprüche 1 bis 5, dadurch gekennzeichnet, dass als starke Säure für die Cyclisierung eine starke Brönsted-Säure verwendet wird.

7. Verfahren nach einem der Patentansprüche 1 bis 6, dadurch gekennzeichnet, dass die Cyclisierung in einem unpolaren Lösungsmittel bei einer Temperatur zwischen -10°C und 70 °C durchgeführt wird.

## Claims (Claims for the following Contracting State(s) : AT, BE, CH, LI, DE, DK, SE, FR, GB, IE, IT, LU, NL, PT)

1. Substituted pentaalkyl chromanes of the general formula: 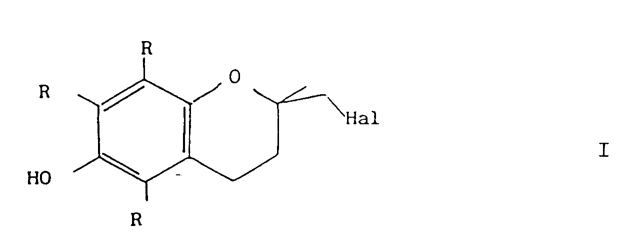 wherein R is a lower alkyl group having 1 to 4 carbon atoms, and Hal is chlorine, bromine or iodine.

2. 2-Chloromethyl-6-hydroxy-2,5,7,8-tetramethyl chromane of the formula:

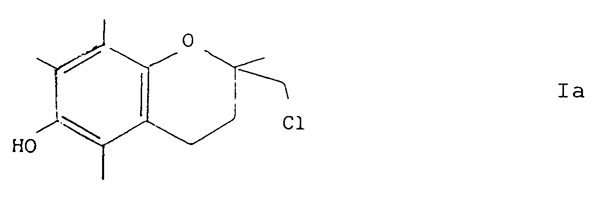

3. A process for the preparation of substituted pentaalkyl chromanes of the general formula: 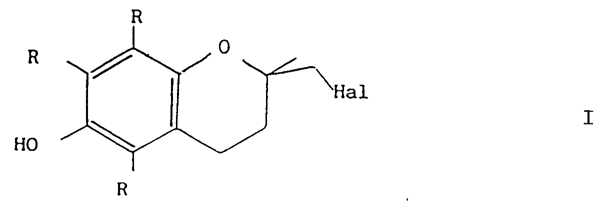 wherein R is a lower alkyl group having 1 to 4 carbon atoms, and Hal is chlorine, bromine or iodine; characterized in that a trialkyl hydroquinone of the general formula: 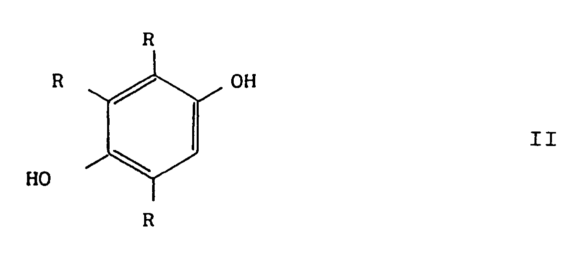 wherein R has the above meaning; is reacted with a halogenated butenol of the formula: 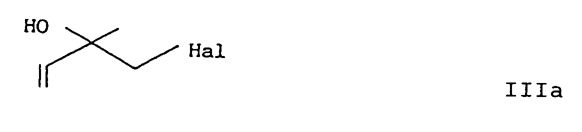 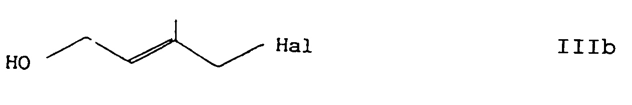 wherein Hal has the above meaning; in the presence of a Lewis acid and in a manner producing a tetraalkyl hydroquinone of the general formula: 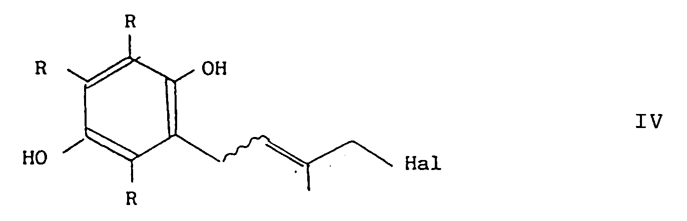 wherein R and Hal have the above meaning; the resulting tetraalkyl hydroquinone being subsequently cyclized to the final product in the presence of a strong acid.

4. The process according to Claim 3, characterized in that there is used as a trialkyl hydroquinone of general formula II the trimethyl derivative wherein R = CH₃, and as a halogenated butenol derivative the 1-chloro-2-methylbut-3-en-2-ol of formula Ilia wherein Hal = Cl.

5. The process according to Claim 3 or 4, characterized in that boron trifluoride or one of its complex compounds is used as a Lewis acid.

6. The process according to any one of Claims 3 to 5, characterized in that the reaction producing tetraalkyl hydroquinone is conducted at a temperature between -10°C and 50 °C in the presence of an inert solvent.

7. The process according to any one of Claims 3 to 6, characterized in that the reaction producing tetraalkyl hydroquinone is conducted in a manner wherein, as soon as the starting trialkyl hydroquinone is practically no longer detectable in the reaction mixture, reaction is stopped and the resulting tetraalkyl hydroquinone is isolated.

8. The process according to any one of Claims 3 to 7, characterized in that a strong Broensted acid is used as a strong acid for cyclization.

9. The process according to any one of Claims 3 to 8, characterized in that cyclization is conducted in a non-polar solvent at a temperature between -10°C and 70 _{°} C.

10. Substituted tetraalkyl hydroquinones of the general formula: 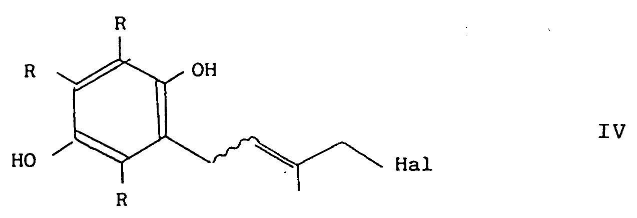 wherein R and Hal have the above meaning.

11. 2-(1-Chloro-2-methylbut-2-en-4-yl)-1,4-dihydroxy-3,5,6-trimethyl benzene: 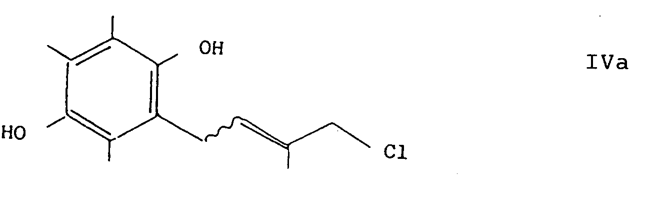 Claims for the following Contracting States : ES, GR

1. A process for the preparation of substituted pentaalkyl chromanes of the general formula: 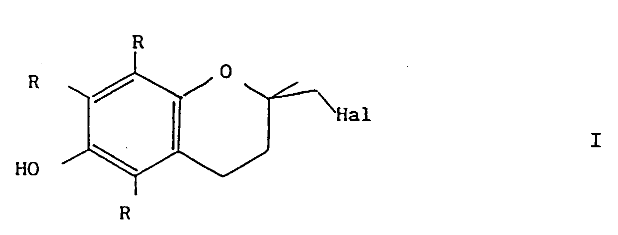 wherein R is a lower alkyl group having 1 to 4 carbon atoms, and Hal is chlorine, bromine or iodine; characterized in that a trialkyl hydroquinone of the general formula: 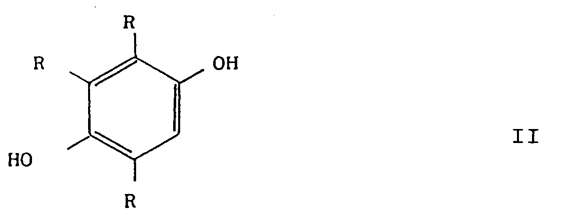 wherein R has the above meaning; is reacted with a halogenated butenol of the formula: 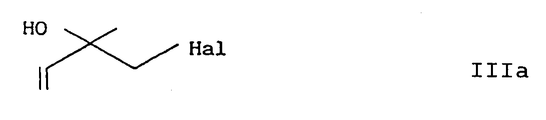 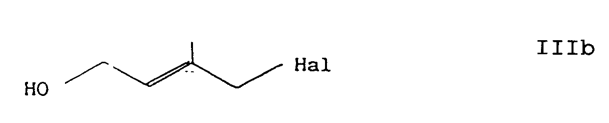 wherein Hal has the above meaning; in the presence of a Lewis acid and in a manner producing a tetraalkyl hydroquinone of the general formula: 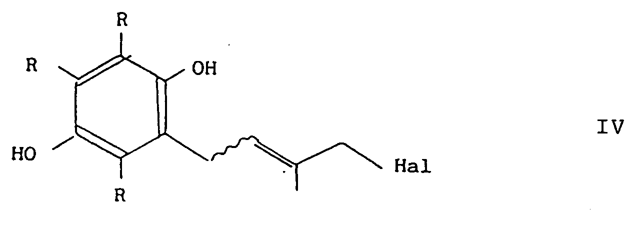 wherein R and Hal have the above meaning; the resulting tetraalkyl hydroquinone being subsequently cyclized to the final product in the presence of a strong acid.

2. The process according to Claim 1, characterized in that there is used as a trialkyl hydroquinone of general formula II the trimethyl derivative wherein R = CH₃, and as a halogenated butenol derivative the 1-chloro-2-methylbut-3-en-2-ol of formula Ilia wherein Hal = Cl.

3. The process according to Claim 1 or 2, characterized in that boron trifluoride or one of its complex compounds is used as a Lewis acid.

4. The process according to any one of Claims 1 to 3, characterized in that the reaction producing tetraalkyl hydroquinone is conducted at a temperature between -10°C and 50 °C in the presence of an inert solvent.

5. The process according to any one of Claims 1 to 4, characterized in that the reaction producing tetraalkyl hydroquinone is conducted in a manner wherein, as soon as the starting trialkyl hydroquinone is practically no longer detectable in the reaction mixture, reaction is stopped and the resulting tetraalkyl hydroquinone is isolated.

6. The process according to any one of Claims 1 to 5, characterized in that a strong Broensted acid is used as a strong acid for cyclization.

7. The process according to any one of Claims 1 to 6, characterized in that cyclization is conducted in a non-polar solvent at a temperature between -10 _{°} C and 70 ° C.

## Revendications (Revendications pour l'(les) Etat(s) contractant(s) suivant(s) : AT, BE, CH, LI, DE, DK, FR, GB, IE, IT, LU, NL, PT, SE)

1. Pentaalkylchromanes substitués de formule générale 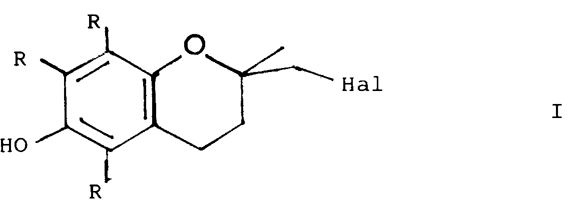 dans laquelle R représente un groupe alkyle inférieur avec 1 à 4 atomes de C et Hal représente le chlore, le brome ou l'iode.

2. 2-chlorométhyl-6-hydroxy-2,5,7,8-tétraméthylchromane de formule

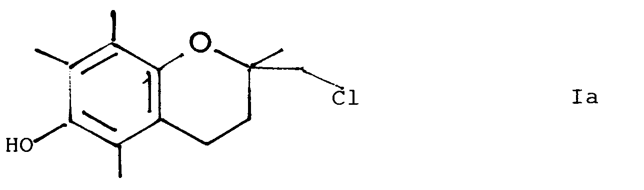

3. Procédé de préparation de pentaalkylchromanes substitués de formule générale 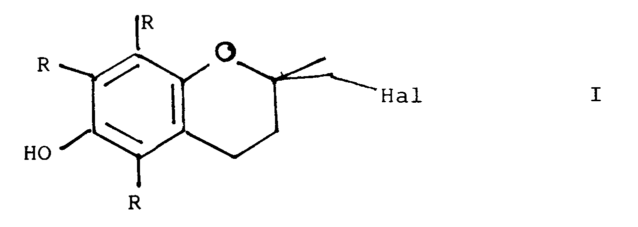 dans laquelle R représente un groupe alkyle inférieur avec 1 à 4 atomes de C et Hal représente le chlore, le brome ou l'iode, caractérisé en ce que l'on fait réagir une trialkylhydroquinone de formule générale 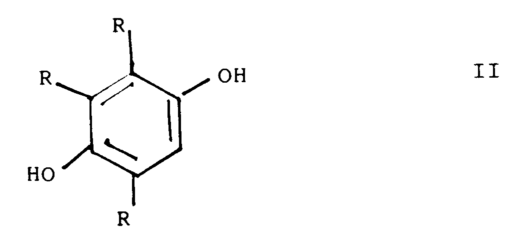 dans laquelle R a la signification précitée, avec un buténol halogéné de formule 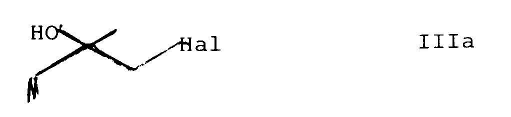 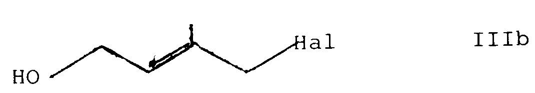 où Hal a la signification indiquée, en présence d'un acide de Lewis de telle manière qu'il en résulte une tétraalkylhydroquinone de formule générale 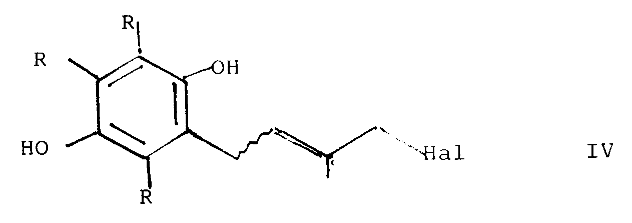 dans laquelle R et Hal possèdent la signification indiquée et que la tétraalkylhydroquinone est finalement cyclisée en le produit final en présence d'un acide fort.

4. Procédé selon la revendication 3, caractérisé en ce qu'on met en oeuvre en tant que trialkylhydroquinone de formule générale Il le dérivé triméthylé avec R = CH₃ et en tant que dérivé de buténol halogéné le 1-chloro-2-méthyl-but-3-ène-2-ol de formule Illa avec Hal = CI.

5. Procédé selon revendication 3 ou 4, caractérisé en ce que du trifluorure de bore ou un de ses complexes est utilisé comme acide de Lewis.

6. Procédé selon l'une des revendications 3 à 5, caractérisé en ce que la transformation en tétraalkylhydroquinone s'effectue à une température entre -10°C et 50 _{°} C en présence d'un solvant inerte.

7. Procédé selon l'une des revendications 3 à 6, caractérisé en ce que la transformation en tétraalkylhydroquinone est conduite de telle manière qu'aussitôt après, lorsque le produit de départ, la trialkylhydroquinone, n'est pratiquement plus décelable dans le mélange réactionnel, la réaction est stoppée et la tétraalkylhydroquinone résultante est isolée.

8. Procédé selon l'une des revendications 3 à 7, caractérisé en ce qu'un acide de Brönsted fort est utilisé comme acide fort pour la cyclisation.

9. Procédé selon l'une des revendications 3 à 8, caractérisé en ce que la cyclisation est réalisée dans un solvant non polaire à une température entre -10°C et 70°C.

10. Tétraalkylhydroquinones substituées de formule générale 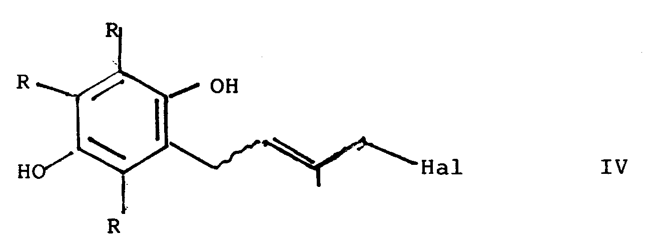 dans laquelle R et Hal possèdent la signification indiquée.

11. 2-(1-chloro-2-méthylbut-2-ène-4-yl)-1,4-dihydroxy-3,5,6-triméthylbenzène

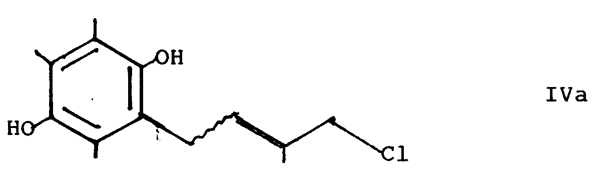

## Revendications (Revendications pour l'(les) Etat(s) contractant(s) suivant(s) : ES, GR)

1. Procédé de préparation de pentaalkylchromanes substitués de formule générale 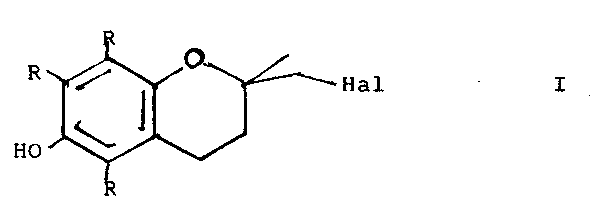 dans laquelle R représente un groupe alkyle inférieur avec 1 à 4 atomes de C et Hal représente le chlore, le brome ou l'iode, caractérisé en ce que l'on fait réagir une trialkylhydroquinone de formule générale 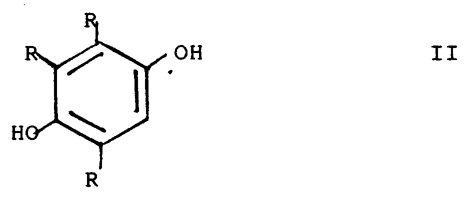 dans laquelle R a la signification précitée, avec un buténol halogéné de formule 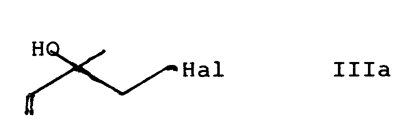 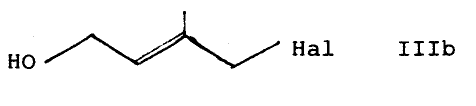 où Hal a la signification indiquée, en présence d'un acide de Lewis de telle manière qu'il en résulte une tétraalkylhydroquinone de formule générale 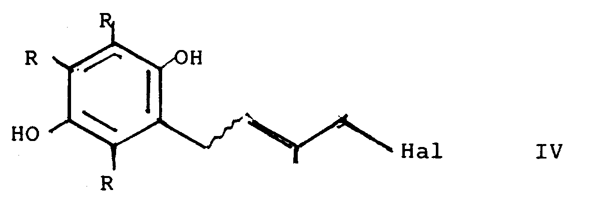 dans laquelle R et Hal possèdent la signification indiquée et que la tétraalkylhydroquinone est finalement cyclisée en le produit final en présence d'un acide fort.

2. Procédé selon la revendication 1, caractérisé en ce qu'on met en oeuvre en tant que trialkylhydroquinone de formule générale Il le dérivé triméthylé avec R = CH₃ et en tant que dérivé de buténol halogéné le 1-chloro-2-méthyl-but-3-ène-2-ol de formule Illa avec Hal = CI.

3. Procédé selon revendication 1 ou 2, caractérisé en ce que du trifluorure de bore ou un de ses complexes est utilisé comme acide de Lewis.

4. Procédé selon l'une des revendications 1 à 3, caractérisé en ce que la transformation en tétraalkylhydroquinone s'effectue à une température entre -10°C et 50°C en présence d'un solvant inerte.

5. Procédé selon l'une des revendications 1 à 4, caractérisé en ce que la transformation en tétraalkylhydroquinone est conduite de telle manière qu'aussitôt après, lorsque le produit de départ, la trialkylhydroquinone, n'est pratiquement plus décelable dans le mélange réactionnel, la réaction est stoppée et la tétraalkylhydroquinone résultante est isolée.

6. Procédé selon l'une des revendications 1 à 5, caractérisé en ce qu'un acide de Brönsted fort est utilisé comme acide fort pour la cyclisation.

7. Procédé selon l'une des revendications 1 à 6, caractérisé en ce que la cyclisation est réalisée dans un solvant non polaire à une température entre -10°C et 70 ° C.
